# EUROPEAN PATENT SPECIFICATION

(11) **EP 0 749 737 B1**
(45) Date of publication and mention of the grant of the patent: **24.11.1999**
(21) Application number: 95120495.7
(22) Date of filing: 22.12.1995
(51) Int. Cl.: A61F 13/15

(54) **Sanitary articles with dual layer topsheet having a selected distribution of large apertures**
Zweifache perforierte Decklage für absorbierende Artikel mit definierter Verteilung der grossen Perforationen
Couche supérieure double avec perforations, pour les articles absorbantes, avec une distribution sélectionnée de perforations

(30) Priority: 19.06.1995 EP 95830255
(43) Date of publication of application: 27.12.1996
(73) Proprietor: THE PROCTER & GAMBLE COMPANY, Cincinnati, Ohio 45202 (US)
(72) Inventor: Divo, Michael, D-61381 Friedrichsdorf (DE); Coles, Peter, I-66023 Francavilla al Mare Chieti (IT); Fornasari, Giancarlo, Pescara (IT)
(74) Representative: Hirsch, Uwe Thomas

(56) References cited:
- EP-A- 0 059 506
- EP-A- 0 165 807
- EP-A- 0 203 821
- EP-A- 0 205 286
- EP-A- 0 545 423
- GB-A- 2 258 840
- US-A- 3 929 135
- US-A- 3 994 771
- US-A- 4 324 246
- US-A- 4 342 314
- US-A- 4 463 045
- US-A- 4 609 518
- US-A- 4 629 643

## Description

### Field of the invention

The present invention relates to absorbent articles particularly sanitary napkins having layered topsheets see EP-A-0 545 423. In particular a first layer receiving the liquids to be absorbed comprises apertures of at least 1.4 mm² area in a film within the longitudinal center of the film. A second layer provides a liquid receiving volume reservoir by having and resiliently maintaining a minimum thickness of 0.3 mm. The apertures in the first layer preferably are also only provided in the transverse center of the film. The distribution of the apertures leaves a certain longitudinal side edge and preferably transverse end edge of the topsheet free of the large apertures to improve comfort of the product during use.

### Background of the invention

Sanitary articles such as sanitary napkins, baby diapers, absorbent inserts, and absorbent adult incontinence articles are well-known in the art. Typically all these articles comprise a wearer facing surface ("internal") and a garment facing surface ("external"). The wearer facing surface receives from the wearer of such articles liquids, bodily discharges such as menses, to be absorbed. In order for the article to store the liquid the wearer facing surface has to be liquid permeable while maintaining integrity of the outer wearer facing surface of the absorbent article. This wearer facing surface is provided by a topsheet and is the one which comes into contact with the wearer's skin.

Well-known topsheets in the art of absorbent articles are non-woven fabrics, woven fabrics or films. Films have to be rendered permeable by aperturing. Fabrics or non-woven fabrics are made of fibers which by their nature provide non-linear apertures of varying and changing size depending on the selected direction for liquid transport through them. Films are often made of polymeric material and typically comprise apertures which have been engineered to provide certain characteristics. These apertures can vary in shape and size. The walls of the apertures define the amount of extension - if any - beyond the plane of the film thickness and the direction of such extensions. The film apertures also can be provided in the shape of a funnel.

A typical topsheet made of polyethylene film has been successfully used in sanitary articles and adult incontinence products as well as inserts and baby diapers. One problem remaining is the total amount of liquid capable of passing through such a topsheet under usual usage conditions due to the total amount of open area of all apertures and individual aperture size and shape in particular. Exceptionally large apertures increase the liquid passage rate but pose the problem of masking because liquids such as menses remain visible to the wearer, which is considered undesirable. Also large apertures promote a backflow of absorbed liquid, so called rewet, which is undesirable. Small individual apertures on the other hand cannot provide the liquid passage characteristics required to let liquids of high surface tensions pass through; this can be a problem in an absolute sense for very small apertures or cause too low a rate of liquid flow.

It also has been found that the total amount of open area for a given aperture size and shape is approximately linearly related to the rate of liquid passage. Again, masking of the liquid which has passed through but also material strength and other appearance considerations are limiting the extent as to which the total open area in a film topsheet can be selected.

Another problem which now has been recognized is that the presence of large hole apertures in the longitudinal side margins or the transverse end regions of the wearer facing surface of topsheet can cause undesirable chaffing or irritation. This is believed to be possibly due to the amount of material forming the rim of the aperture being more for larger apertures than for smaller apertures. It is well-known that even tiny points of irritation from such an article will cause significant discomfort. In fact wearers claiming to experience discomfort typically will disregard any absorbency benefits obtained by the multi aperture topsheet due to unacceptable comfort performance of such articles. This problem is typically accentuated for articles having wings or side wrapping elements.

It is therefore on objective of the present invention to provide a topsheet for sanitary articles which provide the benefits or large apertures in the topsheet while eliminating the problems associated with having large apertures in a region where relative movement between the topsheet and the skin of a wearer would potentially cause discomfort.

From the foresaid it is also clear that a balancing problem between masking, material strength, other appearance considerations and total open area as well as individual aperture size and shape exists in the state of the art. In one aspect the present invention does not attempt to provide selection criteria for this balancing problem but to shift the balance for this problem in order to obtain improved absorbent articles in respect to their capability of fast liquid intake as well as rewet and masking of the liquid received while maintaining acceptable characteristics of material strength and other considerations for topsheets.

It is hence on objective of the present invention to provide an apertured topsheet for absorbent articles which has larger apertures than those commonly found acceptable in film apertured topsheets while effectively improving or at least not deteriorating the rewet and masking of absorbed liquids and providing a distribution of the large apertures eliminating discomfort occurances.

### Description of the invention

The present invention provides an absorbent article according to claim 1 or 3, having all the benefits of a large aperture film topsheet without the comfort, masking, and rewet problems of the prior art. In particular, the absorbent article comprises a laminate topsheet having a wearer facing surface and a garment facing surface. The topsheet comprises a first and second layer which are preferably joined to each other. An absorbent article generally further comprises a backsheet and an absorbent structure alternatively called absorbent core placed between the topsheet and the backsheet.

Two types of absorbent articles have to be distinguished according to the present invention: those having wings or side wrapping elements which comprise at least the first layer of the topsheet and those which do not. The wings or side wrapping elements which are preferred as such can generally be provided by extension of the backsheet, the topsheet, one or several layers of the topsheet or the backsheet, or combinations thereof. Alternatively, wings or side wrapping elements can be provided by materials separately joined to the article. Wings or side wrapping elements are folded along a bending axis around the side edge of the leg opening of an undergarment. Hence the wearer facing surface of the topsheet is most decisive in providing wearer comfort because it is as intinitly in contact with the wearer's skin as the side edge of the undergarment.

### Absorbent structure

The absorbent structure can include the following components: (a) optionally a primary fluid distribution layer preferably together with a secondary optional fluid distribution layer; (b) a fluid storage layer; (c) optionally a fibrous ("dusting") layer underlying the storage layer; and (d) other optional components.

### (a) Primary/Secondary Fluid Distribution Layer

One optional component of the absorbent structure according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers. In particular fibrous layers maintain the capillaries between fibers and even when wet are useful as distribution layers.

### (b) Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", "hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralized, polymeric gelling material. This material forms a hydrogel upon contact with water. Such polymer materials can be prepared from polymerizable, unsaturated, acid-containing monomers which are well-know in the art.

Suitable carriers include materials which are conventionally utilized in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins/panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bonds. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogeneous, i.e. have a distribution gradient in one or several directions within the dimensions of the storage layer. Non-homogeneous distributions can also refer to laminates of carriers enclosing absorbent gelling materials partially or fully.

### (c) Optional Fibrous ("Dusting") Layer

An optional component for inclusion in the absorbent structure according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent structure. Indeed, in those instances where the absorbent gelling material is in the form of macro-structures such as fibers, sheets or strips, this fibrous "dusting" layer need not to be included. However, this "dusting" layer provides some additional fluid-handling capabilities such as rapid wicking of fluid along the length of the pad.

### (d) Other Optional Components of the absorbent structure

The absorbent structure according to the present invention can include other optional components normally present in absorbent webs. For example, a reinforcing scrim can be positioned within the respective layers, or between the respective layers, of the absorbent structure. Such reinforcing scrims should be of such configuration as not to form interfacial barriers to fluid transfer. Given the structural integrity that usually occurs as a result of thermal bonding, reinforcing scrims are usually not required for thermally bonded absorbent structures.

Another component which can be included in the absorbent structure according to the invention and preferably is provided close to or as part of the primary or secondary fluid distribution layer are odor control agents. Active carbon coated with or in addition to other odor control agents, in particular suitable zeolite or clay materials, are optionally incorporated in the absorbent structure. These components can be incorporated in any desired form but often are included as discrete particles.

### Backsheet

The backsheet primarily prevents the exudates absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or non-woven material, polymeric films such as thermoplastic films of polyethylene or polypropylene, or composite materials such as a film-coated non-woven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further, the backsheet can permit vapors to escape from the absorbent structure, i.e. be breathable, while still preventing exudates from passing through the backsheet. Also breathable backsheets comprising several layers, e.g. film plus non-woven structures, can be used.

### The topsheet

The term "joined", as used herein, encompasses configurations in which the first layer is directly secured to the second layer by affixing the first layer directly to the second layer; configurations in which the first layer is indirectly secured to the second layer by affixing the first layer to intermediate layer(s) which in turn is (are) affixed to the second layer. Both layers are preferably joined to each other across at least 25 % of their total interface.

The layers of the topsheet can be joined together by adhesives, stitching, heat and/or pressure bonds, dynamic mechanical bonds, ultrasonic bonds, intermingling or entanglement of the fibers and other structural elements comprising the layers of the topsheet, such as by meltblowing the fibers of the second layer onto the film of the first layer, extruding one layer onto another, or by any other means known in the art.

The topsheet comprises a first passage layer which provides the user facing surface of the topsheet and a second passage layer between the first passage layer and the absorbent structure.

The topsheet as a whole and hence each layer individually needs to be compliant, soft feeling, and non-irritating to the wearer's skin. It also can have elastic characteristics allowing it to be stretched in one or two directions. The topsheet or each of its layers separately extends across the whole of the absorbent structure and can extend into and form part of or all of the preferred wings or side wrapping elements of the absorbent article according to the present invention.

The first passage layer is provided by a film material having apertures which herein are referred to as "large apertures" and optionally apertures which herein are referred to as "small apertures". These apertures are provided to facilitate liquid transport from the wearer facing surface towards the absorbent structure.

For all measurements regarding the apertures in the first passage layer the plane of the smallest cross sectional areas of the aperture should be used, unless otherwise mentioned.

The large apertures have an individual open area of from 1.4 mm², to 3.0 mm² and preferably from 1.5 mm² to 2.5 mm². The total open area of the large apertures in the upper passage layer excluding all other liquid transport apertures should be in the range from 5% to 20%, preferably from 10% to 20% of the surface area of the first layer of the topsheet.

The optional small apertures in the first layer of the topsheet of the absorbent article have an individual open area of less than 1.4 mm² and typically not smaller than 0.15 mm². Apertures which are even smaller are usually not suitable for liquid transport at all and would only function as gas permeable apertures for example for breathability purpose. Preferably, the optional small apertures are in the range of 0.25 mm² to 0.4 mm².

The apertures are preferably substantially circular or polygonal. Their shape is limited by having a ratio of the largest to the smallest inner diagonal length in the range between 1 and 6, preferably 1 and 3. The total open area of all liquid transport apertures in the first layer is in the range of 10% to 40%, preferably 15% to 35% of the total area of the first layer.

Topsheets according to the present invention have a non-homogeneous distribution of the liquid passage ways or apertures across the wearer facing surface of the topsheet. It recently has been found that a topsheet comprising large apertures has a higher tendency of being considered to cause discomfort to some wearers of sanitary napkins comprising such topsheets.

While not wishing to be bound by theory it is considered that the following is an explanation of the reasons for the benefits obtained with the present invention. When creating apertures, e.g. by a thermic process the material initially filling the aperture is not eliminated and ultimately it is collected in the rim around the aperture. This rim becomes more apparent to a wearer the larger the apertures are since the open area of an aperture increases faster than the length of the aperture rim with increasing aperture diameters. The additional material deposited in the rim of the aperture hence builds up more in larger apertures than in smaller apertures. However, with increasing rim material the rim also becomes harsher and harder. Therefore the rim of larger apertures is more prone to cause irritation to the wearer of an article comprising such an apertured topsheet than for small apertures.

The absorbent article and all main components have a longitudinal axis which is parallel to a plane which would bisect a wearer of such an article into right and left side. Also the absorbent article has a transverse axis which is perpendicular to the longitudinal axis and within the same plane as the article. The term "longitudinal center" as used herein refers to the center of the article and extends parallel to the longitudinal axis. The term "transverse center" as used herein refers to the center of the article and extends parallel to the transverse axis.

It hence has been found that a preferable distribution of the apertures should leave the longitudinal margins of the topsheet free of large apertures. Hence the topsheet according to the present invention requires that the large liquid transport apertures mentioned above are only distributed in the longitudinal center 90 % of the largest transverse width of the first passage layer of the topsheet for articles not having wings or side wrapping elements which comprise integral extensions of said first passage layer of said topsheet. Preferably, these large apertures are distributed in the longitudinal center 80 %, most preferably 70 % of the largest transverse width of the first passage layer of the topsheet for articles not having wings or side wrapping elements which comprise integral extensions of said first passage layer of said topsheet.

For articles having wings or side wrapping elements which comprise integral extensions of the first passage layer of said topsheet it is necessary according to the present invention that no large apertures are placed in the vicinity of the bending axis of the wing or side wrapping element. For articles having wings or side wrapping elements the first layer of the topsheet according to the present invention can extend into the wings or side wrapping elements. In such a case a distance of 5 %, preferably 10 %, of the smallest transverse distance between said bending axes, should be left void of large liquid transport apertures around each of the bending axes of each wing or each side wrapping element.

Optionally for ease of manufacturing of the apertured topsheet it is possible to only provide large apertures in the region of the longitudinal center 95 %, preferably 85 %, most preferably 70 %, of the smallest transverse distance between the bending axes of the wings or side wrapping elements to satisfy the more specific requirement stated above according to which large apertures in the first layer of the topsheet are possible even if that layer is integral with wings or side wrapping elements.

In a similar consideration it is also preferable that an embodiment of the present invention has the large apertures only distributed in the transverse center 80 %, preferably 65 %, most preferably 50 % of the largest longitudinal length of the wearer facing surface of the article.

An additional benefit obtainable from the basic or preferred embodiments according to the present invention is the concentration of large liquid transport apertures towards the center of the absorbent article such that more open area is placed where liquid acquisition is most probable. This then provides a benefit in absorbency of the respective absorbent article.

It is apparent to those skilled in the art that the large liquid transport aperture distribution towards the center of the article can be achieved by providing the respective aperture distribution in the first film layer of the topsheet by selective aperturing in particular if the first layer has generally the same extension as the article. An alternative can be of course to only provide the first film layer of the topsheet in the area where large liquid transport apertures are desired with the second layer of the topsheet providing the outer, wearer contacting layer of the topsheet outside that area. This is possible for both articles with or without wings or side wrapping elements which comprise integral extensions of the first layer.

Commonly liquid transport apertures can be formed in the film such that the walls of the apertures extend beyond the plane of the surface of the basic film, i.e. the film surface, before the film is apertured. The direction of these extending walls in the absorbent article is towards the garment facing surface of the article. The amount of extension of the walls of the apertures in the first layer is at least 0.3 mm beyond the film surface form which the walls of the apertures depend. Preferably the walls of the apertures form funnels or Venturi channels as is well-known in the art.

To ensure material stability the smallest distance between neighbouring large apertures regardless of their particular shape and size is preferably at least 1.0 mm, more preferably 1.5 mm. This distance is measured on the surface of the film on the side closest to the user facing surface of the absorbent article.

Also, as is typical for topsheets, the film material is preferably rendered hydrophilic to such a degree that the contact angle is less than 90° with distilled water upon first contact with the water. For films this can be achieved by surfactant treatment. For surfactant treated polymeric films providing the first layer it has been found that it is beneficial to use films where the surfactant is permanently fixed on the film surface. These are so-called film materials with resin integrated surfactant. For these films even repeated wetting by distilled water would provide approximately the same contact angle as the first contact with distilled water.

In another preferred execution of the first layer of the topsheet the wearer facing surface is treated with an agent such that liquids are directed towards the apertures. Such agents can be silicon or teflon which provide the treated surface with a self-cleaning effect. This treatment can be in addition to the above mentioned surfactant treatment.

Films such as those disclosed in EP-A-O 205 286, EP-A-0-165 208, EP-A-0-18 020, EP-A-0-59 506 or US-A-3,929,135 are explicitly referred to as suitable as first passage layer of the topsheet provided the requirements of the claims are met. Other suitable formed films, provided the requirements for the first passage layer are met, are described in U.S. Patent 4,324,246, U.S. Patent 4,342,314, U.S. Patent 4,463,045 and U.S. Patent 5,006,394. Particularly preferred microaperturing of formed film is disclosed in U.S. patent 4,609,518 and U.S. patent 4,629,643. These microapertures can also be included in the first passage layer of the topsheet provided they are less than 0.15 mm² and hence essentially provide breathability. Ways of making such films are well-known in the art and have also been disclosed in the above prior art references.

The second passage layer of the topsheet is provided by a high loft resilient fibrous material. The high loft resilient fibrous material should have a total void volume of more than 50%, preferably more than 90%, preferably a minimum thickness of 0.3 mm, more preferably at least 0.5 mm. The basis weight of such fibrous materials preferably is in the range of 20 g/m² to 100 g/m² and most preferably be in the range of 25 g/m² to 60 g/m².

It is necessary that the fibrous material is at least as hydrophilic as the film material of the first layer in order to present no barrier for the liquid. Better yet it is more hydrophilic (or less hydrophobic) than the film and creates a directing force for the liquid towards the absorbent structure after passing the first layer. Hence if the fibers are made of synthetic fibers such as polypropylene, polyethylene, bicomponent polypropylene/polyethylene, polyester, polyamid fibers or artificial fibers preferably rayon, viscose fibers or mixtures thereof, it may be necessary to treat these fibers in a similar fashion as the film in order to provide them with the required hydrophilic (or hydrophobic) characteristic.

The fibers in this second layer should preferably be thermally bonded in order to provide integrity and resiliency to the network of fibrous material desired under the present invention. The fibrous layer should be resilient in the sense that it returns to at least 80% of its original volume after being compressed to 50% of the starting volume in a dry state. In a wet state the layer should return to at least 65% of its original volume after being compressed to 50% of the starting volume.

For carded fibrous materials the individual fiber length should be preferably from 25 mm to 50 mm, while the preferred air-laid fibers are preferably 1 o 10 mm, most preferably 4 o 8 mm long. Fibers should have a weight per length in the range from 1.5 to 10 x 10⁻⁷ kg/m, preferably 3.3 to 6.7 x 10⁻⁷ kg/m (decitex).

Fibrous layers are well-known in the art of absorbent articles to provide topsheets. Such fibrous layers, provided the requirements as indicated in the claims are met, can be used as the second layer in the context of the present invention. In particular fibrous layers in accordance with US-Application 08/141,156 of October 21, 1993 or US-A-3,994,771 are useful as the second layer of the topsheet according to the present invention.

## Claims

1. **.** Absorbent article, having enhanced liquid intake, rewet, masking, and comfort performance, said article having a longitudinal axis and a transverse axis and said article comprising three main elements a topsheet, a backsheet, and an absorbent structure placed between said topsheet and said backsheet, said article and each of said main elements having a wearer facing surface and a garment facing surface, and said topsheet comprising
- a first passage layer, said first passage layer providing said wearer facing surface of said topsheet, and
- a second passage layer, said second passage layer being placed between said first passage layer and said absorbent structure,
- said article not comprising wings or side wrapping elements which comprise integral extensions of said first passage layer of said topsheet,
- both said passage layers being preferably joined to each other,
- said first passage layer being provided by a film material having large liquid transport apertures,
- said large liquid transport apertures having an individual open area in the range from 1.4 mm² to 3.0 mm²,
- said large liquid transport apertures have a total open area in the range of from 5% to 20% of the total area of said first passage layer,
- said large liquid transport apertures following a distribution such that said large liquid transport apertures are only distributed in the longitudinal center 90 % of the largest transverse width of the first passage layer of said topsheet,
- said liquid transport apertures having a largest inner diagonal length and a smallest inner diagonal length, the ratio of said largest to said smallest inner diagonal length being in the range of from 1 to 6,
- said liquid transport apertures preferably having walls which depend at least 0.3 mm from the surface of said film, said walls depending in a direction towards said garment facing surface,
- said film material preferably being rendered hydrophilic such that it forms a contact angle of less than 90 degrees with distilled water upon first contact with distilled water,
- the second passage layer being provided by a high loft fibrous material,
- said fibrous material having a void volume of more than 50%, and a thickness of at least 0.3 mm,
- said fibrous material being at least as hydrophilic as said film material of said first passage layer.

2. Absorbent article according to claim 1 wherein said large liquid transport apertures are only distributed in the longitudinal center 80 %, preferably 70 %, of said largest transverse width of said first passage layer of said topsheet.

3. Absorbent article, having enhanced liquid intake, rewet, masking, and comfort performance, said article having a longitudinal axis and a transverse axis and said article comprising three main elements a topsheet, a backsheet, and an absorbent structure placed between said topsheet and said backsheet, said article and each of said main elements having a wearer facing surface and a garment facing surface, said article comprising wings or side wrapping elements and which wings or side wrapping elements can each be folded along a bending axis, and said topsheet comprising
- a first passage layer, said first passage layer providing said wearer facing surface of said topsheet, and integrally forming at least part of said wings or side wrapping elements, and
- a second passage layer, said second passage layer being placed between said first passage layer and said absorbent structure,
- both said passage layers being preferably joined to each other,
- said first passage layer being provided by a film material having large liquid transport apertures,
- said large liquid transport apertures having an individual open area in the range from 1.4 mm² to 3.0 mm²,
- said large liquid transport apertures have a total open area in the range of from 5% to 20% of the total area of said first passage layer,
- said large liquid transport apertures following a distribution such that said large liquid transport apertures are only distributed in the longitudinal center 90 % of the smallest transverse distance between said bending axes of said wings or said side wrapping elements,
- said liquid transport apertures having a largest inner diagonal length and a smallest inner diagonal length, the ratio of said largest to said smallest inner diagonal length being in the range of from 1 to 6,
- said liquid transport apertures preferably having walls which depend at least 0.3 mm from the surface of said film, said walls depending in a direction towards said garment facing surface,
- said film material preferably being rendered hydrophilic such that it forms a contact angle of less than 90 degrees with distilled water upon first contact with distilled water,
- the second passage layer being provided by a high loft fibrous material,
- said fibrous material having a void volume of more than 50%, and a thickness of at least 0.3 mm,
- said fibrous material being at least as hydrophilic as said film material of said first passage layer.

4. Absorbent article according to claim 3 wherein said large liquid transport apertures are only distributed in the longitudinal center 85 %, preferably 70 %, of said largest transverse distance between said bending axes of said wings or said side wrapping elements.

5. Absorbent article according to any of the preceding claims wherein said first passage layer is provided by a film material further having small apertures for liquid transport,
- said small apertures having an individual open area in the range of from 0.15 mm² to less than 1.4 mm²,
- the total open area of all said liquid transport apertures in said first passage layer being in the range of from 10% to 40% of the total area of said first passage layer.

6. Absorbent article according to claim 5 wherein said small apertures have an individual open area in the range of from 0.25 mm² to 0.4 mm².

7. Absorbent article according to any of the preceding claims wherein said large liquid transport apertures are only distributed in the transverse center 80 %, preferably 65 %, most preferably 50 %, of the largest longitudinal length of said wearer facing surface of said article.

8. Absorbent article according to any of the preceding claims wherein said fibrous material has a void volume of more than 90%.

9. Absorbent article according to any of the preceding claims wherein said fibrous material has a basis weight of from 20 g/m² to 100 g/m², preferably form 25 g/m² to 60g/m².

10. Absorbent article according to any of the preceding claims wherein both said first and said second passage layer are joined directly to each other.

## Patentansprüche

1. Absorbierender Artikel mit erhöhter Flüssigkeitsaufnahme-, Wiederbenetzungs-, Verdeckungs- und Komfortleistung, wobei der genannte Artikel eine Längsachse und eine Querachse umfaßt, und der genannte Artikel drei Hauptelemente, ein Deckblatt, ein Rückenblatt und eine absorbierende Struktur, welche zwischen dem genannten Deckblatt und dem genannten Rückenblatt angeordnet ist, umfaßt, wobei der genannte Artikel und jedes der genannten Hauptelemente eine dem Träger zugewandte Oberfläche und eine der Kleidung zugewandte Oberfläche aufweisen und das genannte Deckblatt umfaßt
- eine erste Durchgangsschichte, wobei die genannte erste Durchgangsschichte die genannte dem Träger zugewandte Oberfläche des genannten Deckblatts beistellt, und
- eine zweite Durchgangsschichte, wobei die genannte zweite Durchgangsschichte zwischen der genannten ersten Durchgangsschichte und der genannten absorbierenden Struktur angeordnet ist,
- der genannte Artikel nicht Flügel oder seitenumhüllende Elemente umfaßt, welche integrale Verlängerungen der genannten ersten Durchgangsschichte des genannten Deckblatts umfassen,
- wobei beide genannten Durchgangsschichten vorzugsweise miteinander verbunden sind,
- die genannte erste Durchgangsschichte durch ein Folienmaterial beigestellt ist, welches große Flüssigkeitstransportöffnungen aufweist,
- die genannten großen Flüssigkeitstransportöffnungen eine individuelle offene Fläche im Bereich von 1,4 mm² bis 3,0 mm² aufweisen,
- die genannten großen Flüssigkeitstransportöffungen eine gesamte offene Fläche im Bereich von 5 % bis 20 % der Gesamtfläche der genannten ersten Durchgangsschichte aufweisen,
- die genannten großen Flüssigkeitstransportöffungen einer Verteilung derart folgen, daß die genannten großen Flüssigkeitstransportöffnungen nur in der Längsmitte über 90 % der breitesten Querbreite der ersten Durchgangsschichte des genannten Deckblatts verteilt sind,
- die genannten Flüssigkeitstransportöffnungen eine größte innere Diagonallänge und eine kleinste innere Diagonallänge aufweisen, wobei das Verhältnis der genannten größten zur genannten kleinsten inneren Diagonallänge im Bereich von 1:6 ist,
- die genannten Flüssigkeitstransportöffnungen vorzugsweise Wandungen aufweisen, welche von der Oberfläche der genannten Folie mindestens 0,3 mm herabhängen, wobei die genannten Wandungen in einer Richtung gegen die genannte der Kleidung zugewandte Oberfläche herabhängen,
- das genannte Folienmaterial vorzugsweise hydrophil gemacht ist, sodaß es beim ersten Kontakt mit destilliertem Wasser einen Kontaktwinkel von weniger als 90° mit destilliertem Wasser bildet,
- die zweite Durchgangsschichte durch ein faseriges Hochbauschmaterial beigestellt ist,
- das genannte faserige Material ein Hohlraumvolumen von mehr als 50 % und eine Dicke von mindestens 0,3 mm aufweist,
- das genannte faserige Material mindestens so hydrophil wie das genannte Folienmaterial der genannten ersten Durchgangsschichte ist.

2. Absorbierender Artikel nach Anspruch 1, bei welchem die genannten großen Flüssigkeitstransportöffnungen lediglich in der Längsmitte über 80 %, vorzugsweise über 70 % der genannten breitesten Querbreite der genannten ersten Durchgangsschichte des genannten Deckblatts verteilt sind.

3. Absorbierender Artikel mit erhöhter Flüssigkeitsaufnahme-, Wiederbenetzungs-, Verdeckungs- und Komfortleistung, wobei der genannte Artikel eine Längsachse und eine Querachse aufweist und der genannte Artikel drei Hauptelemente, ein Deckblatt, ein Rückenblatt und eine absorbierende Struktur, welche zwischen dem genannten Deckblatt und dem genannten Rückenblatt angeordnet ist, umfaßt, wobei der genannte Artikel und jedes der genannten Hauptelemente eine dem Träger zugewandte Oberfläche und eine der Kleidung zugewandte Oberfläche aufweisen, der genannte Artikel Flügel oder seitenumhüllende Elemente umfaßt, und welche Flügel oder seitenumhüllenden Elemente jeweils entlang einer Biegeachse gefaltet werden können, und das genannte Deckblatt umfaßt
- eine erste Durchgangsschichte, wobei die genannte erste Durchgangsschichte die genannte dem Träger zugewandte Oberfläche des genannten Deckblatts beistellt und integral mindestens einen Teil der genannten Flügel oder der seitenumhüllenden Elemente bildet, und
- eine zweite Durchgangsschichte, wobei die genannte zweite Durchgangsschichte zwischen der genannten ersten Durchgangsschichte und der genannten absorbierenden Struktur angeordnet ist,
- wobei beide genannten Durchgangsschichten vorzugsweise miteinander verbunden sind,
- die genannte erste Durchgangsschichte von einem Folienmaterial beigestellt ist, welches große Flüssigkeitstransportöffnungen aufweist,
- die genannten großen Flüssigkeitstransportöffnungen eine individuelle offene Fläche im Bereich von 1,4 mm² bis 3,0 mm² aufweisen,
- die genannten großen Flüssigkeitstransportöffnungen eine gesamte offene Fläche im Bereich von 5 % bis 20 % der Gesamtfläche der genannten ersten Durchgangsschichte aufweisen,
- die genannten großen Flüssigkeitstransportöffnungen einer Verteilung so folgen, daß die genannten großen Flüssigkeitstransportöffnungen lediglich in der Längsmitte über 90 % der kleinsten Querdistanz zwischen den genannten Biegeachsen der genannten Flügel oder der genannten seitenumhüllenden Elemente verteilt sind,
- die genannten Flüssigkeitstransportöffnungen eine größte innere Diagonallänge und eine kleinste innere Diagonallänge aufweisen, wobei das Verhältnis der genannten größten zur genannten kleinsten inneren Diagonallänge im Bereich von 1 bis 6 liegt,
- die genannten Flüssigkeitstransportöffnungen vorzugsweise Wandungen aufweisen, welche von der Oberfläche der genannten Folie mindestens 0,3 mm herabhängen, wobei die genannten Wandungen in einer Richtung gegen die genannte der Kleidung zugewandte Oberfläche herabhängen,
- das genanne Folienmaterial vorzugsweise so hydrophil gemacht ist, daß es beim ersten Kontakt mit destilliertem Wasser einen Kontaktwinkel von weniger als 90° mit destilliertem Wasser bildet,
- die zweite Durchgangsschichte von einem faserigen Hochbauschmaterial beigestellt ist,
- das genannte faserige Material ein Hohlraumvolumen von mehr als 50 % und eine Dicke von mindestens 0,3 mm aufweist,
- das genannte faserige Material mindestens so hydrophil wie das genannte Folienmaterial der genannten ersten Durchgangsschichte ist.

4. Absorbierender Artikel nach Anspruch 3, bei welchem die genannten großen Flüssigkeitstransportöffnungen lediglich in der Längsmitte über 85 %, vorzugsweise über 70 % der genannten breitesten Querdistanz zwischen den genannten Biegeachsen der genannten Flügel oder der genannten seitenumhüllenden Elemente verteilt sind.

5. Absorbierender Artikel nach einem der vorhergehenden Ansprüche, bei welchem die genannte erste Durchgangsschichte von einem Folienmaterial beigestellt ist, welches weiters kleine Öffnungen für Flüssigkeitstransport aufweist,
- wobei die genannten kleinen Öffnungen eine individuelle offene Fläche im Bereich von 0,15 mm² bis weniger als 1,4 mm² aufweisen,
- die gesamte offene Fläche aller genannten flüssigkeitstransportierendern Öffnungen in der genannten ersten Durchgangsschichte im Bereich von 10 % bis 40 % der gesamten Fläche der genannten ersten Durchgangsschichte ist.

6. Absorbierender Artikel nach Anspruch 5, bei welchem die genannten kleinen Öffnungen eine individuelle offene Fläche im Bereich von 0,25 mm² bis 0,4 mm² aufweisen.

7. Absorbierender Artikel nach einem der vorhergehenden Ansprüche, bei welchem die genannten großen Flüssigkeitstransportöffnungen lediglich in der Quermitte über 80 %, vorzugsweise über 65%, am bevorzugtesten über 50 % der größten Längslänge der genannten dem Träger zugewandten Oberfläche des genannten Artikels verteilt sind.

8. Absorbierender Artikel nach einem der vorhergehenden Ansprüche, bei welchem das genannte faserige Material ein Hohlraumvolumen von mehr als 90 % aufweist.

9. Absorbierender Artikel nach einem der vorhergehenden Ansprüche, bei welchem das genannte faserige Material ein Flächengewicht von 20g/m² bis 100g/m², vorzugsweise von 25g/m² bis 60g/m² aufweist.

10. Absorbierender Artikel nach einem der vorhergehenden Ansprüche, bei welchem sowohl die genannte erste wie auch die genannte zweite Durchgangsschichte direkt miteinander verbunden sind.

## Revendications

1. Article absorbant, ayant une performance améliorée d'admission de liquide, de remouillage, de masquage et de confort, ledit article ayant un axe longitudinal et un axe transversal, et ledit article comprenant trois éléments principaux, une feuille de dessus, une feuille de fond et une structure absorbante placée entre ladite feuille de dessus et ladite feuille de fond, ledit article et chacun desdits éléments principaux ayant une surface faisant face à l'utilisateur et une surface faisant face au vêtement, et ladite feuille de dessus comprenant :
- une première couche de passage, ladite première couche de passage fournissant ladite surface faisant face à l'utilisateur de ladite feuille de dessus, et
- une deuxième couche de passage, ladite deuxième couche de passage étant placée entre ladite première couche de passage et ladite structure absorbante,
- ledit article ne comprenant pas d'ailes ou d'éléments enveloppants latéraux qui comprennent des prolongements solidaires de ladite première couche de passage de ladite feuille de dessus;
- les deux dites couches de passage étant réunies, de préférence, l'une à l'autre,
- ladite première couche de passage étant réalisée avec un matériau pelliculaire présentant de grands orifices de transport de liquide,
- lesdits grands orifices de transport de liquide ayant une surface ouverte individuelle comprise dans la plage allant de 1,4 mm² à 3,0 mm²,
- lesdits grands orifices de transport de liquide ont une surface ouverte totale comprise dans la plage allant de 5% à 20% de la surface totale de ladite première couche de passage,
- lesdits grands orifices de transport de liquide suivant une répartition telle que lesdits grands orifices de transport de liquide ne sont répartis que dans les 90% de la partie centrale longitudinale de la largeur transversale la plus grande de la première couche de passage de ladite feuille de dessus,
- lesdits orifices de transport de liquide ayant une longueur diagonale intérieure la plus grande et une longueur diagonale intérieure la plus petite, le rapport entre lesdites longueurs diagonales intérieures la plus grande et la plus petite étant compris dans une plage allant de 1 à 6,
- lesdits orifices de transport de liquide ayant, de préférence, des parois qui pendent au moins de 0,3 mm par rapport à la surface dudit film, lesdites parois pendant dans une direction orientée vers ladite surface faisant face au vêtement,
- ledit matériau pelliculaire étant, de préférence, rendu hydrophile de façon à former un angle de contact inférieur à 90 degrés avec de l'eau distillée lors du premier contact avec de l'eau distillée,
- la deuxième couche de passage étant réalisée avec un matériau fibreux à bouffant élevé,
- ledit matériau fibreux ayant un volume de vide supérieur à 50% et une épaisseur d'au moins 0,3 mm.
- ledit matériau fibreux étant au moins aussi hydrophile que ledit matériau pelliculaire de ladite première couche de passage.

2. Article absorbant selon la revendication 1, dans lequel lesdits grands orifices de transport de liquide ne sont répartis que dans les 80% de la partie centrale longitudinale, de préférence 70%, de ladite largeur transversale la plus grande de ladite première couche de passage de ladite feuille de dessus.

3. Article absorbant, ayant une performance améliorée d'admission de liquide, de remouillage, de masquage et de confort, ledit article ayant un axe longitudinal et un axe transversal, et ledit article comprenant trois éléments principaux, une feuille de dessus, une feuille de fond et une structure absorbante placée entre ladite feuille de dessus et ladite feuille de fond, ledit article et chacun desdits éléments principaux ayant une surface faisant face à l'utilisateur et une surface faisant face au vêtement, ledit article comprenant des ailes ou des éléments enveloppants latéraux, et lesquelles ailes ou lesquels éléments enveloppants latéraux peuvent, chacun, être pliés le long d'un axe de cintrage, et ladite feuille de dessus comprenant :
- une première couche de passage, ladite première couche de passage fournissant ladite surface faisant face à l'utilisateur de ladite feuille de dessus, et formant de mariière solidaire au moins une partie desdites ailes ou desdits éléments enveloppants latéraux, et
- une deuxième couche de passage, ladite deuxième couche de passage étant placée entre ladite première couche de passage et ladite structure absorbante,
- les deux dites couches de passage étant réunies, de préférence, l'une à l'autre,
- ladite première couche de passage étant réalisée avec un matériau pelliculaire présentant de grands orifices de transport de liquide,
- lesdits grands orifices de transport de liquide ayant une surface ouverte individuelle comprise dans la plage allant de 1,4 mm² à 3,0 mm²,
- lesdits grands orifices de transport de liquide ont une surface ouverte totale comprise dans la plage allant de 5% à 20% de la surface totale de ladite première couche de passage,
- lesdits grands orifices de transport de liquide suivant une répartition telle que lesdits grands orifices de transport de liquide ne sont répartis que dans les 90% de la partie centrale longitudinale de la distance transversale la plus petite séparant lesdits axes de cintrage desdites ailes ou desdits éléments enveloppants latéraux,
- lesdits orifices de transport de liquide ayant une longueur diagonale intérieure la plus grande et une longueur diagonale intérieure la plus petite, le rapport entre lesdites longueurs diagonales intérieures la plus grande et la plus petite étant compris dans une plage allant de 1 à 6,
- lesdits orifices de transport de liquide ayant, de préférence, des parois qui pendent au moins de 0,3 mm par rapport à la surface dudit film, lesdites parois pendant dans une direction orientée vers ladite surface faisant face au vêtement,
- ledit matériau pelliculaire étant, de préférence, rendu hydrophile de façon à former un angle de contact inférieur à 90 degrés avec de l'eau distillée lors du premier contact avec de l'eau distillée,
- la deuxième couche de passage étant réalisée avec un matériau fibreux à bouffant élevé,
- ledit matériau fibreux ayant un volume de vide supérieur à 50% et une épaisseur d'au moins 0,3 mm.
- ledit matériau fibreux étant au moins aussi hydrophile que ledit matériau pelliculaire de ladite première couche de passage.

4. Article absorbant selon la revendication 3, dans lequel lesdits grands orifices de transport de liquide ne sont répartis que dans les 85% de la partie centrale longitudinale, de préférence 70%, de ladite distance transversale la plus grande séparant lesdits axes de cintrage desdites ailes ou desdits éléments enveloppants latéraux.

5. Article absorbant selon l'une quelconque des revendications précédentes, dans lequel ladite première couche de passage est réalisée avec un matériau pelliculaire ayant, en outre, de petits orifices pour le transport de liquide,
- lesdits petits orifices ayant une surface ouverte individuelle comprise dans la plage allant de 0,15 mm² à moins de 1,4 mm²,
- la surface ouverte totale de tous lesdits orifices de transport de liquide ménagés dans ladite première couche de passage étant comprise dans la plage allant de 10% à 40% de la surface totale de ladite première couche de passage.

6. Article absorbant selon la revendication 5, dans lequel lesdits petits orifices ont une surface ouverte individuelle comprise dans la plage allant de 0,25 mm² à 0,4 mm².

7. Article absorbant selon l'une quelconque des revendications précédentes, dans lequel lesdits grands orifices de transport de liquide ne sont répartis que dans les 80% de la partie centrale transversale, de préférence 65%, de la manière la plus préférée 50%, de la longueur longitudinale la plus grande de ladite surface faisant face à l'utilisateur dudit article.

8. Article absorbant selon l'une quelconque des revendications précédentes, dans lequel ledit matériau fibreux a un volume de vide supérieur à 90%.

9. Article absorbant selon l'une quelconque des revendications précédentes, dans lequel ledit matériau fibreux a un grammage compris entre 20 g/m² et 100 g/m², de préférence entre 25 g/m² et 60 g/m².

10. Article absorbant selon l'une quelconque des revendications précédentes, dans lequel les deux dites première et deuxième couches de passage sont réunies directement l'une à l'autre.
